# EUROPÄISCHE PATENTANMELDUNG

(11) **EP 0 827 726 A2**
(43) Veröffentlichungstag der Anmeldung: **11.03.1998**
(21) Anmeldenummer: 97890176.7
(22) Anmeldetag: 04.09.1997
(51) Int. Cl.: A61F 2/30, A61L 27/00

(54) **Implantat, insbesondere Gelenk-Prothesenimplantat**

(30) Priorität: 04.09.1996 AT 1575/96; 18.07.1997 AT 1232/97
(71) Anmelder: Implantech Medizintechnik Ges.m.b.H., 2344 Maria Enzersdorf (AT)
(72) Erfinder: Wurzinger, Anton, Dr., 2344 Maria Enzersdorf (AT)
(74) Vertreter: Kopecky, Helmut, Dipl.-Ing. Kopecky & Schwarz Patentanwälte

(57) **Zusammenfassung**

Bei einem Implantat mit einer strukturierten Oberfläche an den mit einem Knochen in Kontakt gelangenden Flächen ist zwecks Erzielung einer gezielten Porosität die strukturierte Oberfläche wie folgt gestaltet:

Die strukturierte Oberfläche ist mit einer Vielzahl zylindrischer Vertiefungen (1), insbesondere Bohrungen, mit Durchmessern von 0,3 bis 1 mm, vorzugsweise 0,5 bis 1 mm, und mit Tiefen zwischen 0,3 und 1 mm, vorzugsweise 0,5 und 1 mm, sowie gegebenenfalls zylindrischen Erhebungen (2) versehen, so daß eine Porosität zwischen 50 und 80 %, vorzugsweise 60 und 70 %, bewirkt ist (Fig. 2).

## Beschreibung

Die Erfindung betrifft ein Implantat, insbesondere Gelenk-Prothesenimplantat, mit knochenseitigen strukturierten Oberflächen.

Vor allem bei einer zementlosen Implantation spielt die Osseointegration eine besondere Rolle, um die Stabilität des Implantates über einen längeren Zeitraum zu gewährleisten. Es ist daher erforderlich, daß die mit der Knochenstruktur kontaktierende Oberfläche Eigenschaften aufweist, die den Knochen anregen, durch Aufbau von neuen Knochenstrukturen an diese Oberfläche heran- und hineinzuwachsen. Da sich glatte Flächen als untauglich erwiesen haben, hat man bereits vorgeschlagen, die Oberfläche des Implantats an der Berührungsfläche mit der Knochenstruktur porös zu gestalten bzw. aufzurauhen, also gitterförmig, pyramidenförmig, kassettenförmig u. dgl. zu gestalten. Man hat auch bereits vorgeschlagen, auf diese Oberfläche Kugeln aufzusintern. Sinterprozesse können jedoch ungünstige Materialeigenschaften hervorrufen. Weiters besteht die Gefahr, daß sich Kugeln ablösen.

Es ist auch bereits bekannt, die Oberflächen der Implantate im Spritzgußverfahren herzustellen. Die so gebildeten Oberflächen weisen eine ausgezeichnete Aufrauhung auf. Nachteilig ist jedoch, daß sich die Porosität nicht exakt bestimmen läßt. Vielmehr sind die Erhöhungen und Vertiefungen willkürlich über die Oberfläche verteilt und nicht den Anforderungen angepaßt. Sind die Aufrauhungen zu flach, ist die Porosität für eine gute Osseointegration zu gering, sind die Aufrauhungen zu stark ausgeprägt, leidet die Festigkeit darunter. In diesem Fall stehen erhöhtes Porenvolumen und Festigkeit in einem ungünstigen Verhältnis zueinander. Weiters können gebirgsartige Vorsprünge abbrechen und es können die Verbindungsbrücken geschwächt werden. Dies alles führt dazu, daß die Oberfläche für eine gute Osseointegration nicht optimal ist.

Aus der US-A 5 489 306 ist ein für die Einsetzung in den Hohlraum eines langen Knochens vorgesehenes Implantat bekannt, welches mehrere poröse Zonen mit unterschiedlicher Porengröße aufweist, derart, daß die Porengröße vom proximalen Bereich zum distalen Bereich abnimmt. Ein derartiges Implantat ist nur für den Einsatz in langen Knochen verwendbar.

Die Erfindung bezweckt die Vermeidung dieser Nachteile und Schwierigkeiten und stellt sich die Aufgabe, ein Implantat der eingangs beschriebenen Art zu schaffen, dessen strukturierte Oberflächen genau eine gewünschte, für das Osseointegrationsverhalten optimale Porosität Oberfläche in einem günstigen Verhältnis zum Volumen der vorgesehenen Struktur der Oberfläche steht.

Diese Aufgabe wird erfindungsgemäß dadurch gelöst, daß die strukturierte Oberfläche mit einer Vielzahl zylindrischer Vertiefungen, insbesondere Bohrungen, mit Durchmessern von 0,3 bis 1 mm, vorzugsweise 0,5 bis 1 mm, und mit Tiefen zwischen 0,3 und 1 mm, vorzugsweise 0,5 und 1 mm, versehen ist, so daß eine Porosität zwischen 50 und 80 %, vorzugsweise 60 und 70 %, bewirkt ist.

Gemäß einer bevorzugten Ausführungsform sind zusätzlich zu den zylindrischen Vertiefungen zylindrische Erhebungen mit Durchmessern zwischen 0,3 und 1 mm, vorzugsweise 0,5 und 1 mm, vorhanden, wobei zweckmäßig das Verhältnis der Anzahl der zylindrischen Erhebungen zur Anzahl der zylindrischen Vertiefungen 3 : 7 beträgt, und wobei ebenfalls eine Porosität zwischen 50 und 80 %, vorzugsweise 60 und 70 % vorhanden ist. Diese Erhöhungen bewirken in der Frühphase nach der Implantation und vor erfolgter, abgeschlossener Osseointegration eine zusätzliche Stabilität zum Knochen und schalten vor allem Mikrobewegungen aus, die durch Scherkräfte verursacht werden könnten.

Bevorzugt sind die zylindrischen Vertiefungen sowie gegebenenfalls die zylindrischen Erhebungen an ebenen knochenseitigen Flächen des Implantats vorgesehen und an den nicht-planen knochenseitigen Flächen kraterförmig strukturierte Vertiefungen mit Tiefen bis zu 0,5 mm vorgesehen.

Eine bevorzugte Ausführungsform ist dadurch gekennzeichnet, daß maximal 40 % der Oberfläche Poren mit einem Randdurchmesser zwischen 100 µm und 300 µm, maximal 40 % der Oberfläche Poren mit einem Randdurchmesser zwischen 300 µm und 500 µm und maximal 80 % der Oberfläche Poren mit einem Randdurchmesser zwischen 500 µm und 800 µm aufweisen. Diese exakt definierte und abgestimmte Porenstruktur provoziert, wie Versuche gezeigt haben, den Knochen zu einer sukzessiven und raschen Osseointegration. Dabei steht die Stabilität und Belastbarkeit der Oberfläche in einem wesentlich günstigeren Verhältnis zum Porenvolumen als bei den bekannten Implantaten. Durch die kontrollierte und zugleich optimierte, höchstporöse Oberfläche wird gleichzeitig auch ein höheres Maß an Sicherheit erreicht. Die bei den Oberflächenstrukturen der bekannten Implantate mit erhöhter Porosität entstehenden Risken sind bei dem erfindungsgemäß ausgebildeten Implantat wesentlich reduziert.

Optimale Ergebnisse werden erzielt, wenn die Tiefe der Poren das einfache bis zweieinhalbfache des Randdurchmessers beträgt und der kleinste Abstand zwischen den Rändern benachbarter Poren zwischen 100 µm und 400 µm beträgt.

Das erfindungsgemäße Implantat kann eine von Poren bedeckte Oberfläche zwischen 50 % und 80 % der die Knochensubstanz kontaktierenden Gesamtoberfläche aufweisen, wodurch sich eine optimale Stabilität und Belastbarkeit dieser Oberfläche ergibt.

Weiters hat es sich als zweckmäßig erwiesen, wenn die Fläche der Poren, also jene Fläche, die vom Porenrand ausgehend in der Oberfläche nach innen ragt, abgerundet ausgebildet ist, also keine scharfen Ecken und Kanten aufweist, durch welche die Osseointegration ungünstig beeinflußt wird.

Erfindungsgemäß können zwischen den Poren Erhöhungen vorgesehen sein, deren Höhe zweckmäßig maximal 1000 µm beträgt.

Bei Versuchen hat sich das erfindungsgemäße Implantat sehr bewährt, wenn die Oberfläche aus einem Reintitan, einer Titan-Guß- oder Schmiede-Legierung oder eine Kobalt-Chrom-Legierung besteht. Dadurch wird sichergestellt, daß die mit den Poren versehene Oberfläche eine gute Korrosionsbeständigkeit und Körperverträglichkeit besitzt.

Eine Optimierung kann weiters dadurch erzielt werden, daß die Oberfläche mit einer zusätzlichen Beschichtung, vorzugsweise mit einer Dicke von etwa 20 µm, versehen ist. Insbesondere bei Verwendung einer Kobalt-Chrom-Legierung hat sich eine Beschichtung aus Reintitan als zweckmäßig erwiesen. Es kann aber auch die Rauhigkeit und Anwuchsfreudigkeit des Knochens durch eine Beschichtung aus einer der Knochenstruktur ähnlichen, mineralischen Substanz, insbesondere aus Hydroxylapatit, verbessert werden.

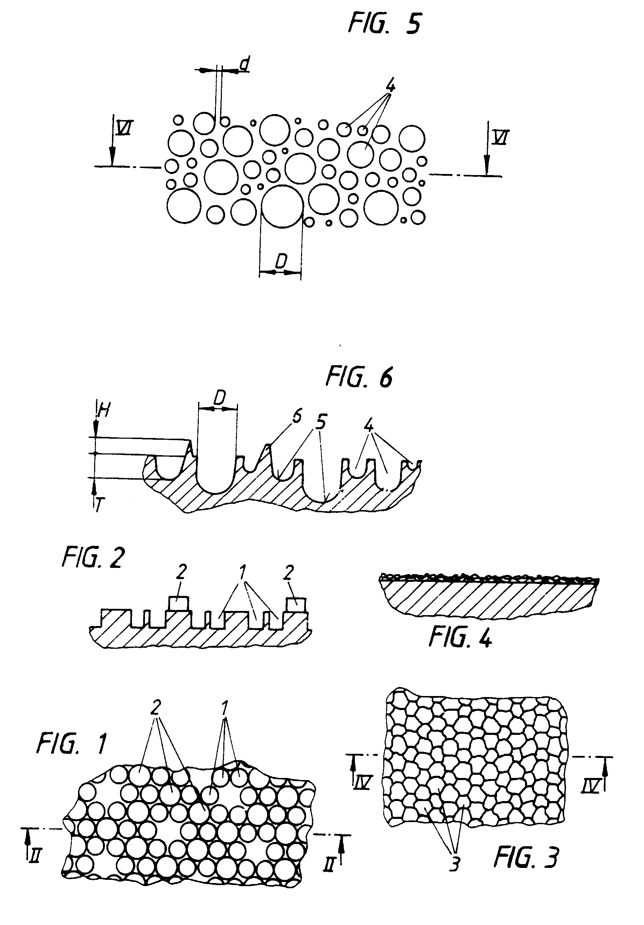

Die Erfindung ist nachstehend anhand von Ausführungsbeispielen näher erläutert, wobei Fig. 1 eine Draufsicht in Vergrößerung auf eine ebene, mit dem Knochen in Kontakt gelangende Fläche eines Implantats veranschaulicht. Fig. 2 ist eine Schnittdarstellung eines nach der Linie II-II der Fig. 1 geführten Schnittes. Fig. 3 ist eine Draufsicht auf eine nicht-ebene Fläche eines Implantats und Fig. 4 eine Schnittdarstellung analog zu Fig. 2 nach der Linie IV-IV der Fig. 3. Fig. 5 zeigt in größerem Maßstab eine Draufsicht auf einen Ausschnitt der Oberfläche eines erfindungsgemäßen Implantates, und Fig. 6 stellt einen Längsschnitt nach der Linie VI-VI der Fig. 5 dar.

Die in die Fig. 1 und 2 dargestellte strukturierte Oberfläche ist beispielsweise an einem Gelenk-Prothesenimplantat eines Kniegelenks vorgesehen, u.zw. an einer ebenen Fläche einer am Tibiakopf aufliegenden Platte des Gelenk-Prothesenimplantats. Die Strukturierung wird durch eine Vielzahl zylindrischer Bohrungen 1 mit Durchmessern von 0,3 bis 1 mm, vorzugsweise 0,5 bis 1 mm, und mit Tiefen zwischen 0,3 und 1 mm, vorzugsweise 0,5 und 1 mm erreicht. Weiters sind noch zylindrische Erhebungen 2 vorhanden, u.zw. mit Durchmessern zwischen 0,3 und 1 mm, vorzugsweise 0,5 und 1 mm, und Höhen zwischen 0,3 und 1 mm, vorzugsweise 0,5 und 1 mm, wobei das Verhältnis der Anzahl der zylindrischen Erhebungen 2 zur Anzahl der zylindrischen Bohrungen 1 3 : 7 beträgt. Insgesamt wird hierdurch eine Porosität zwischen 50 und 80, vorzugsweise 60 und 70 %, bewirkt. Als Porosität ist der Prozentsatz des Luftvolumens über einem Oberflächenbereich, der mit Bohrungen bzw. Erhebungen versehen ist, gegenüber dem massiven Volumen dieses Oberflächenbereiches zu verstehen.

An den nicht-planen Flächen, mit denen das Implantat 4 mit dem Knochen in Berührung steht, sind kraterförmige Vertiefungen 3 mit Tiefen bis zu 0,5 mm vorgesehen, wie dies in den Fig. 3 und 4 dargestellt ist.

Das Implantat mit der strukturierten Oberfläche ist aus Metall gebildet; insbesondere können hier vorzugsweise folgende Metalle zum Einsatz gelangen: Rein-Titan, eine Titan-Guß- oder Titan-Schmiede-Legierung oder eine Kobalt-Chrom-Legierung.

Dadurch, daß für die strukturierte Oberfläche Erhebungen 2 und Vertiefungen 1 von genau festgelegter Struktur vorgesehen sind, wobei die Vertiefungen 1 und Erhebungen 2 exakt und einfach herstellbar sind, läßt sich die Porosität genau vorherbestimmen, d.h. es können strukturierte Oberflächen je nach gewünschter Porosität in einfacher Weise hergestellt werden, ohne daß man von Zufälligkeiten beim Herstellungsprozeß abhängig ist, wie dies beim Stand der Technik der Fall ist. Hierdurch gelingt es, für Implantate jeweils ein optimales Osseointegrationsverhalten sicherzustellen. Erfindungsgemäß läßt sich eine sehr hohe Porosität erzielen, ohne daß die Gefahr besteht, daß die Porosität beim Manipulieren des Implantates durch Beschädigung an den Erhebungen und Vertiefungen geringer wird. Dies wird durch die erfindungsgemäße zylindrische Gestaltung der Erhebungen 2 und Vertiefungen 1 erreicht.

Die Oberfläche des in den Fig. 5 und 6 dargestellten Implantates besitzt Poren 4, wobei maximal 40 % der Oberfläche Poren 4 mit einem Randdurchmesser D zwischen 100 µm und 300 um, maximal 40 % der Oberfläche Poren 4 mit einem Randdurchmesser D zwischen 300 µm und 500 µm und maximal 80 % der Oberfläche Poren 4 mit einem Randdurchmesser D zwischen 500 µm aufweisen. Die Tiefe T der Poren 4 beträgt das einfache bis zweieinhalbfache des Randdurchmessers D, und der kleinste Abstand d zwischen den Rändern benachbarter Poren 4 beträgt zwischen 100 µm und 400 µm.

Wie aus Fig. 6 hervorgeht, ist die Bodenfläche 5 der Poren 4 abgerundet. Zwischen den Poren 4 können Erhebungen 6 vorgesehen sein, deren Höhe H maximal 1000 µm beträgt.

Die Oberfläche des Implantates, welche die Poren 4 aufweist, kann aus einem Reintitan, einer Titan-Guß- oder Schmiede-Legierung oder aus einer Kobalt-Chrom-Legierung bestehen und gegebenenfalls mit einer nicht dargestellten zusätzlichen Beschichtung versehen sein, deren Dicke etwa 20 µm beträgt. Diese Beschichtung kann beispielsweise aus Reintitan oder aus einer der Knochenstruktur ähnlichen mineralischen Substanz, insbesondere Hydroxylapatit, bestehen.

## Patentansprüche

1. Implantat, insbesondere Gelenk-Prothesenimplantat, mit knochenseitigen strukturierten Oberflächen, dadurch gekennzeichnet, daß die strukturierte Oberfläche mit einer Vielzahl zylindrischer Vertiefungen (1, 4), insbesondere Bohrungen, mit Durchmessem von 0,3 bis 1 mm, vorzugsweise 0,5 bis 1 mm, und mit Tiefen zwischen 0,3 und 1 mm, vorzugsweise 0,5 und 1 mm, versehen ist, so daß eine Porosität zwischen 50 und 80 %, vorzugsweise 60 und 70 %, bewirkt ist.

2. Implantat nach Anspruch 1, dadurch gekennzeichnet, daß zusätzlich zu den zylindrischen Vertiefungen (1, 4) zylindrische Erhebungen (2, 6) vorhanden sind mit Durchmessern zwischen 0,3 und 1 mm, vorzugsweise 0,5 und 1 mm.

3. Implantat nach Anspruch 2, dadurch gekennzeichnet, daß das Verhältnis der Anzahl der zylindrischen Erhebungen (2, 6) zur Anzahl der zylindrischen Vertiefungen (1, 4) 3 : 7 beträgt, und wobei ebenfalls eine Porosität zwischen 50 und 80 %, vorzugsweise 60 und 70 %, vorhanden ist.

4. Implantat nach Anspruch 1 bis 3, dadurch gekennzeichnet, daß die zylindrischen Vertiefungen (1, 4) sowie gegebenenfalls die zylindrischen Erhebungen (2, 6) an ebenen knochenseitigen Flächen des Implantats vorgesehen sind, und daß an den nicht-planen knochenseitigen Flächen kraterförmig strukturierte Vertiefungen mit Tiefen bis zu 0,5 mm vorgesehen sind.

5. Implantat nach einem oder mehreren der Ansprüche 1 bis 4, dadurch gekennzeichnet, daß maximal 40 % der Oberfläche Poren (4) mit einem Randdurchmesser (D) zwischen 100 µm und 300 µm, maximal 40 % der Oberfläche Poren (4) mit einem Randdurchmesser (D) zwischen 300 µm und 500 µm und maximal 80 % der Oberfläche Poren (4) mit einem Randdurchmesser (D) zwischen 500 µm und 800 µm aufweisen (Fig. 5, 6).

6. Implantat nach Anspruch 5, dadurch gekennzeichnet, daß die Tiefe (T) der Poren (4) das einfache bis zweieinhalbfache des Randdurchmessers (D) beträgt.

7. Implantat nach Anspruch 5 oder 6, dadurch gekennzeichnet, daß der kleinste Abstand zwischen den Rändern benachbarter Poren (4) zwischen 100 µm und 400 µm beträgt.

8. Implantat nach einem oder mehreren der Ansprüche 5 bis 7, dadurch gekennzeichnet, daß die von den Poren bedeckte Oberfläche zwischen 50 % und 80 % der eine Knochensubstanz kontaktierenden Gesamtoberfläche beträgt.

9. Implantat nach einem oder mehreren der Ansprüche 5 bis 8, dadurch gekennzeichnet, daß die Bodenfläche (5) der Poren (4) abgerundet ausgebildet ist.

10. Implantat nach einem oder mehreren der Ansprüche 1 bis 9, dadurch gekennzeichnet, daß die Oberfläche aus einem Reintitan besteht.

11. Implantat nach einem oder mehreren der Ansprüche 1 bis 10, dadurch gekennzeichnet, daß die Oberfläche aus einer Titan-Guß- oder Schmiede-Legierung besteht.

12. Implantat nach einem oder mehreren der Ansprüche 1 bis 11, dadurch gekennzeichnet, daß die Oberfläche aus einer Kobalt-Chrom-Legierung besteht.

13. Implantat nach einem oder mehreren der Ansprüche 1 bis 12, dadurch gekennzeichnet, daß die Oberfläche mit einer zusätzlichen Beschichtung, vorzugsweise mit einer Dicke von etwa 20 um, versehen ist.

14. Implantat nach Anspruch 13, dadurch gekennzeichnet, daß die Beschichtung aus Reintitan besteht.

15. Implantat nach Anspruch 13, dadurch gekennzeichnet, daß die Beschichtung aus einer der Knochenstruktur ähnlichen mineralischen Substanz, insbesondere Hydroxylapatit, besteht.
